# EUROPÄISCHE PATENTSCHRIFT

(11) **EP 1 361 829 B1**
(45) Veröffentlichungstag und Bekanntmachung des Hinweises auf die Patenterteilung: **08.06.2005**
(21) Anmeldenummer: 02712797.6
(22) Anmeldetag: 20.02.2002
(51) Int. Cl.: A61B 19/00

(54) **VORRICHTUNG ZUR STEUERUNG VON CHIRURGISCHEN INSTRUMENTEN**
DEVICE FOR CONTROLLING SURGICAL INSTRUMENTS
DISPOSITIF POUR PILOTER DES INSTRUMENTS CHIRURGICAUX

(30) Priorität: 22.02.2001 DE 10108547
(43) Veröffentlichungstag der Anmeldung: 19.11.2003
(73) Patentinhaber: SIEMENS AKTIENGESELLSCHAFT, 80333 München (DE)
(72) Erfinder: GRAUMANN, Rainer, 91315 Höchstadt (DE)
(86) Internationale Anmeldenummer: PCT/DE2002/000615
(87) Internationale Veröffentlichungsnummer: WO 2002/065931

(56) Entgegenhaltungen:
- WO-A-01/64124
- DE-A- 19 807 884
- DE-A- 19 917 867
- US-A- 4 791 934
- US-A- 5 408 409
- US-A- 5 772 594

## Beschreibung

Die vorliegende Erfindung betrifft ein Operationssystem zum Durchführen von operativen Eingriffen an einem Patienten.

Bei operativen Eingriffen an einem Patienten erfolgt die Steuerung von chirurgischen Instrumenten, wie z.B. Laparoskope, Endoskope, Nadeln, Roboter usw., entweder auf der Basis der direkten Sicht, wie z.B. beim Laparoskop und Endoskop, der pre-operativen Bildgebung, wie z.B. Robotik in der Orthopädie, oder der intra-operativen Bildgebung, z.B. bei der Nadelbiopsie unter Computertomographie- (CT-) und Ultraschall-Kontrolle.

Ein der Präambel des Anspruchs 1 entsprechendes Operationsystem ist in DE-A-198 07 884 beschrieben.

Beispielhaft für einen robotergestützten operativen Eingriff sind Operationen am Knie (beim sog. Total Knee Replacement) und an der Hüfte (beim sog. Total Hip Replacement) zu nennen. Derartige Eingriffe werden beim Stand der Technik ausschließlich unter Verwendung pre-operativer CT-Röntgenbilder durchgeführt, d.h. die Aufnahme des zu operierenden Bereichs erfolgt vor dem operativen Eingriff. Der Nachteil dieses Verfahrens ist jedoch, das es mit einer aufwendigen Registrierungsprozedur verbunden ist.

Andere, komplexere Eingriffe erfordern jedoch laufende Kontrollbilder während des Eingriffs, um eine sichere Durchführung zu gewährleisten. Dazu gehört beispielsweise die Vertebroplastie, bei der eine sterile Substanz (Zement) in brüchig gewordene Wirbelkörper gefüllt wird, um diese Wirbelkörper zu stabilisieren.

Die Aufgabe der vorliegenden Erfindung ist somit, ein Operationssystem zur Durchführung von operativen Eingriffen an einem Patienten und ein in diesem Operationssystem angewendetes Verfahren zur Steuerung von chirurgischen Instrumenten während des Eingriffs bereitzustellen, bei denen eine zuverlässige und exakte Steuerung der chirurgischen Instrumente während des operativen Eingriffs auf der Basis von intra-operativen Röntgenbildern ermöglicht wird.

Diese Aufgabe wird durch ein Operationssystem zum Durchführen von operativen Eingriffen mittels eines chirurgischen Instruments an einem Patienten gemäß dem beigefügten Anspruch 1.

Das erfindungsgemäße Operationssystem zum Durchführen von operativen Eingriffen mittels eines chirurgischen Instruments an einem Patienten beinhaltet ein Röntgengerät zum Aufnehmen eines Röntgenbildes von mindestens einem Bereich (Körperteil, Organ) des Patienten, wobei das aufgenommene Röntgenbild durch eine Ausgabevorrichtung bildlich dargestellt wird, und ein Positionserfassungssystem zum Erfassen der Position des Röntgengerätes während der Aufnahme und zum permanenten Erfassen der Position des chirurgischen Instruments während des operativen Eingriffs.

Die erfassten Positionen werden durch eine Verarbeitungsvorrichtung verarbeitet; die Verarbeitungsvorrichtung blendet dabei kontinuierlich während des operativen Eingriffs eine Darstellung des chirurgischen Instruments in das angezeigte Röntgenbild. Die Einblendung des Röntgenbildes und der Darstellung des chirurgischen Instrumentes erfolgt dabei lagerichtig gemäß der tatsächlichen räumlichen Beziehung zwischen der Position des aufgenommenen Bereichs des Patienten und der Position des chirurgischen Instruments.

Gemäß der vorliegenden Erfindung wird von dem Patienten bzw. von dem Bereich (Körperteil, z.B. Organ), in dem der chirurgische Eingriff erfolgen soll, eine Röntgenaufnahme gemacht und mittels des Positionserfassungssystems die räumliche Position ermittelt, von der die Röntgenaufnahme gemacht wurde. Während des chirurgischen Eingriffs wird durch das Positionserfassungssystem laufend die Position des mindestens einen chirurgischen Instruments ermittelt und eine entsprechende Darstellung (d.h. eine Abbildung des chirurgischen Instruments) in die Anzeige der Röntgenaufnahme eingeblendet. Die Einblendung erfolgt dabei lagerichtig in der relativen Position, in der das chirurgische Instrument aktuell zu dem betroffenen Bereich liegt, d.h. dass das angezeigte räumliche Verhältnis zwischen dem chirurgischen Instrument und der durch das Röntgenbild aufgenommene Körperpartie gleich dem tatsächlichen räumlichen Verhältnis zueinander ist.

Das chirurgische Instrument wird durch einen Roboter oder Manipulator geführt, so dass zum Teil bestimmte Vorgänge automatisch bzw. mit einer wesentlich höheren Präzision als bei einem manuellen Eingriff durchgeführt werden können.

Ein Roboter ermöglicht die automatisierte Durchführung bestimmter Vorgänge, beispielsweise das Ausfräsen bestimmter Bereiche der Hüfte bei einer Hüftoperation, wo genau definierte Bereiche ausgefräst werden.

Ein Manipulator dagegen besteht aus einem Roboterarm, der beispielsweise durch einen Joystick vom Chirurgen geführt wird. Ein Manipulator ermöglicht dem Chirurgen ein wesentlich präziseres Arbeiten als per Hand, da durch den Manipulator beispielsweise die Instrumente aufgrund einer Übersetzung präziser geführt werden können und ggf. Zittern des Chirurgen kompensiert werden kann.

Der Vorteil der vorliegenden Erfindung besteht darin, dass während des chirurgischen Eingriffs laufend Röntgenbilder zur Kontrolle gemacht werden können, um so den Fortschritt des operativen Eingriffs zu verfolgen. Der Verlauf der Operation, der somit auf den Röntgenbildern sichtbar wird, kann damit bei der Führung des chirurgischen Instruments einbezogen werden.

Ein weiterer Vorteil der vorliegenden Erfindung besteht darin, dass einige der bisher manuell durchgeführten Eingriffe am Skelett robotergestützt durchgeführt werden können, da gemäß der vorliegenden Erfindung eine zuverlässige und ausreichende Bildgebung durch Röntgenbilder, beispielsweise von dreidimensionalen Röntgenbildern, während der Operation (d.h. intra-operativ) zur Verfügung steht.

Es wird somit eine Anwendung von Robotern bzw. Manipulatoren in operativen Eingriffen ermöglicht, die eine laufende Kontrolle durch Röntgenbilder erfordern.

Vorteilhafte Ausgestaltungen der vorliegenden Erfindung sind in den jeweiligen Unteransprüchen wiedergegeben.

Das Positionserfassungssystem beinhaltet eine erste Positionsmarkierung, die an dem Röntgengerät angebracht ist, z.B. einem C-Bogen Röntgengerät, eine zweite Positionsmarkierung, die an dem chirurgischen Instrument oder an dem Roboter/Manipulator, der das Instrument führt, angebracht ist, und eine Positionserfassungsvorrichtung zum Erfassen der jeweiligen Positionen von Röntgengerät und dem chirurgischen Instrument aufweist. Dabei ist die räumliche Beziehung zwischen der ersten Positionsmarkierung und der räumlichen Position des Körperteils des Patienten, der durch das Röntgengerät erfasst wird, und die räumliche Beziehung zwischen der zweiten Positionsmarkierung und dem Arbeitsbereich (z.B. Spitze) des chirurgischen Instruments bekannt.

Das Positionserfassungssystem kann beispielsweise auf einem optischen, ein akustischen oder einem elektromagnetischen System basieren.

Zum Einbeziehen z.B. der Patientenbewegung während der Operation wird der Patient bzw. der Bereich des Patienten, der röntgenbildlich erfasst wird, ebenfalls mittels des Positionserfassungssystems erfasst. Dazu wird in der Umgebung des entsprechenden Bereichs ein dritte Positionsmarkierung zum Erfassen der Patientenposition (bzw. der Position des entsprechenden Körperteils) angebracht, wobei die Position des dritten Positionssensors permanent erfasst wird und die Verarbeitungsvorrichtung ggf. Patientenbewegungen in das angezeigte Bild einberechnet. Die dritte Positionsmarkierung muss dabei im Verhältnis zum entsprechenden Bereich (z.B. Knie) fest angebracht sein; hier ist ebenfalls die räumliche Beziehung zwischen dem dritten Positionssensor und dem entsprechenden Körperteil bekannt.

Das erfindungsgemäße Operationssystem beinhaltet bei einem Einsatz eines Roboters eine Steuervorrichtung zur automatischen Steuerung des Roboters bei automatisierten, beispielsweise oben beschriebenen, operativen Eingriffen. Die Steuervorrichtung berücksichtigt dabei den aufgenommenen Röntgendatensatz (bzw. das Röntgenbild), die Position des Bereichs, der mit dem Röntgenbild dargestellt wird und die Position des chirurgischen Instruments.

Bei Einsatz eines Manipulators kann der operative Eingriff durch die Steuervorrichtung automatisch kontrolliert werden, so dass beispielsweise der Manipulator nur einen eingeschränkten oder vorbestimmten Arbeitsbereich ansteuern kann, um unbeabsichtigte Verletzungen des Patienten zu vermeiden.

Die vorliegende Erfindung wird nachfolgend anhand eines bevorzugten Ausführungsbeispieles unter Bezug auf die beigefügten Zeichnungen näher erläutert, in denen zeigen
- Fig. 1: eine schematische Darstellung des Operationssystems gemäß der vorliegenden Erfindung, und
- Fig. 2: ein Funktionsdiagramm, das das Konzept der vorliegenden Erfindung zeigt.

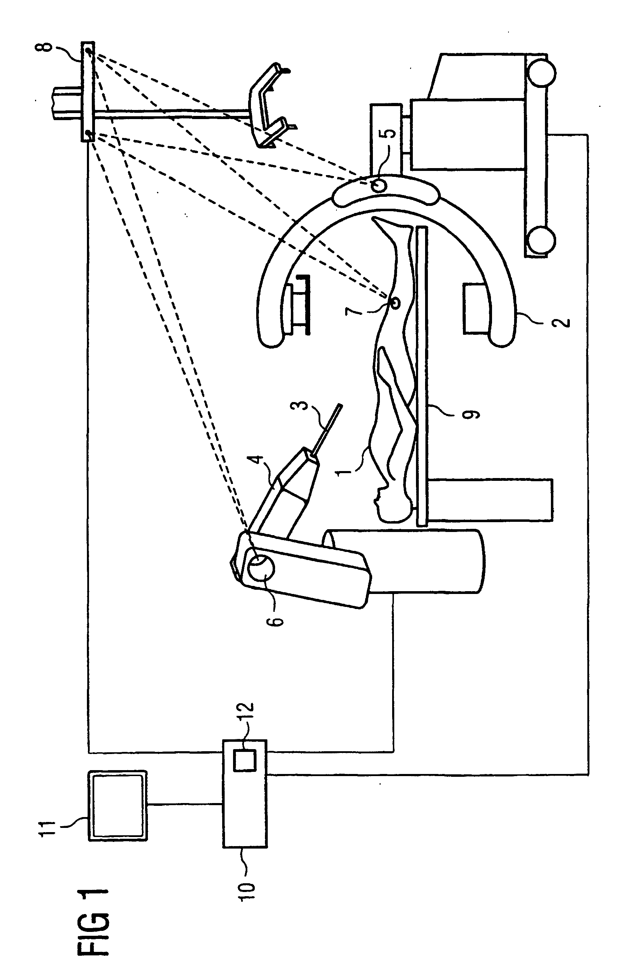

Anhand von Fig. 1 wird nachfolgend das erfindungsgemäße Operationssystem beschrieben.

Das erfindungsgemäße Operationssystem beinhaltet ein Röntgengerät 2, im Beispiel ein mobiles C-Bogen Röntgengerät zur Aufnahme von zwei- und dreidimensionalen Röntgenbildern (bzw. Röntgendatensätzen). Mittels des Röntgengerätes 2 werden zwei- bzw. dreidimensionale Röntgendatensätze beispielsweise vom Knie des Patienten 1, der auf einem Operationstisch 9 liegt, aufgenommen.

Die dreidimensionalen Röntgendatensätze werden gewonnen, indem zuerst durch das Röntgengerät 2 eine Vielzahl von zweidimensionalen Röntgenbildern von einem bestimmten Bereich des Patienten 1 aufgenommen werden. Dabei werden durch den C-Bogen des Röntgengeräts 2 von dem bestimmten Bereich (z.B. Knie) eine Vielzahl von zweidimensionalen Röntgenbildern unter verschiedenen Winkeln aufgenommen. Die so gewonnenen zweidimensionalen Röntgendatensätze werden durch einen Computer unter Anwendung bekannter Projektionsmatrizen in mindestens einen dreidimensionalen Röntgendatensatz projiziert. Auf diese Weise kann ein dreidimensionaler Bereich mit einem bestimmten Volumen aufgenommen und dargestellt werden.

Weiterhin beinhaltet das erfindungsgemäße Operationssystem ein chirurgisches Instrument 3, das durch einen Roboter 4 oder Manipulator geführt wird, und eine Positionserfassungsvorrichtung 8.

Die Positionserfassungsvorrichtung 8 ist Bestandteil des Positionserfassungssystems und dient zur Erfassung der Positionen von dem chirurgischen Instrument 3 und dem Röntgengerät 2. Zu diesem Zweck ist an dem Röntgengerät 2 eine erste Positionsmarkierung 5 und an dem chirurgischen Instrument bzw. dem Roboter 4, der das chirurgische Instrument 3 führt, eine zweite Positionsmarkierung 6 angebracht.

Das erfindungsgemäße Operationssystem beinhaltet außerdem eine Steuervorrichtung 12 zur automatischen Steuerung bzw. Kontrolle des Roboters bzw. Manipulators 4.

Die online Bildgebung erlaubt gemäß der vorliegenden Erfindung die Abbildung des interessierenden Organs direkt im Operationsumfeld, d.h. dass die notwendigen Röntgenbilder während der Operation aufgenommen werden; die Aufnahme von Röntgenbildern vor der Operation (pre-operativ), die mit einer aufwendigen Registrierungsprozedur zur Durchführung des operativen Eingriffs verbunden ist, entfällt somit.

Das Problem, dass Objektverschiebungen, Deformationen, Repositionierungen, usw. des betroffenen Bereichs in der Zeit zwischen der pre-operativen Bildgebung und des operativen Eingriffs bzw. während des operativen Eingriffs auftreten können, wird kompensiert, da durch die intra-operative Bildgebung der betroffene Bereich korrekt und damit geometrisch genau abgebildet wird.

Zur Herstellung der Beziehung zwischen der räumlichen Position des chirurgischen Instruments 3 und der räumlichen Position des Bereichs des Patienten, dessen Röntgenbild zwei- bzw. dreidimensional dargestellt wird, muss die Beziehung zwischen der an dem Roboter 4 bzw. chirurgischen Instrument 3 angebrachten zweiten Positionsmarkierung 6 und dem Arbeitsbereich (z.B. der Spitze) des chirurgischen Instruments 3 bekannt sein.

Weiterhin muss die Beziehung zwischen der an dem Röntgengerät 2 angebrachten ersten Positionsmarkierung 5 und der räumlichen Position des aufgenommenen Röntgenbilds bekannt sein. Diese Beziehung wird ausführlich in der Patentanmeldung "Verfahren zur Ermittlung einer Koordinatentransformation für die Navigation eines Objektes" der Siemens AG mit der Anmelde-Nr. DE 10042963.7 beschrieben.

Durch diese bekannten Beziehungen kann die direkte räumliche Beziehung zwischen dem Röntgenbild (Röntgendatensatz), dem Patienten (bzw. dem entsprechenden Bereich bzw. Organ) und dem chirurgischen Instrument durch die Verarbeitungsvorrichtung 10 hergestellt und auf einem Bildschirm (Anzeigevorrichtung 11) dargestellt werden, so dass das Instrument direkt mittels der Bildkontrolle geführt und gesteuert werden kann. Diese räumlichen Beziehungen können auch von der Steuervorrichtung 12 wie beschrieben zur Steuerung und Kontrolle des Roboters/Manipulators 4 weiterverarbeitet werden.

In einer vorteilhaften Ausgestaltung der vorliegenden Erfindung ist in dem Bereich des Patienten 1, der durch das Röntgengerät erfasst wird (im Beispiel das Knie), ein dritte Positionsmarkierung 7 angebracht. Diese Positionsmarkierung 7 wird ebenfalls durch die Positionserfassungsvorrichtung registriert. Dadurch wird erreicht, dass eventuelle Bewegungen des Patienten während des Eingriffs bei der bildlichen Darstellung des chirurgischen Instruments 3 berücksichtigt werden können, indem diese Bewegungen durch die Verarbeitungsvorrichtung 10 in die Darstellung des Instruments 3 in die Darstellung des Patienten 1 einberechnet und auf dem Bildschirm 11 ausgegeben werden. Auf diese Weise wird gewährleistet, dass das angezeigte aktuelle räumliche Verhältnis zwischen dem aufgenommenen Bereich des Patienten 1 und dem chirurgischen Instrument 3 weiterhin mit dem tatsächlichen räumlichen Verhältnis übereinstimmt.

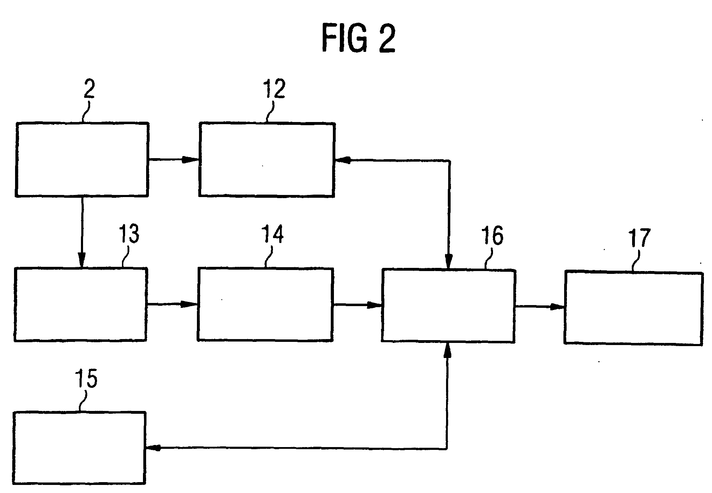

Fig. 2 zeigt ein Funktionsdiagramm, das den Ablauf der Steuerung des chirurgischen Instruments gemäß der vorliegenden Erfindung zeigt.

Das Röntgengerät 2 liefert zum einen zwei- bzw. dreidimensionale Röntgenbilder 12. Zum anderen wird durch das Positionserfassungssystem eine räumliche Position 13 des Röntgengeräts 2 ermittelt. Aus der räumlichen Position 13 des Röntgengeräts (bzw. dessen Positionsmarkierung) wird, z.B. wie in der zitierten Patentanmeldung "Verfahren zur Ermittlung einer Koordinatentransformation für die Navigation eines Objektes", die tatsächliche räumliche Position 14 des Bereiches, der mit dem Röntgenbild bzw. der Darstellung dieses Röntgenbildes erfasst wird, ermittelt.

Weiterhin wird durch das Positionserfassungssystem eine Position 15 des chirurgischen Instruments bzw. des Roboters oder Manipulators, der das chirurgische Instrument führt, erfasst.

Das aufgenommene Röntgenbild 12, die Position 14 des aufgenommenen Bereichs und die Position 15 des chirurgischen Instruments wirken auf die Steuerung 16 bzw. Führung des chirurgischen Instruments (Position 15 des chirurgischen Instruments) und somit auf den operativen Eingriff 17 ein. Die Steuerung 16 des chirurgischen Instruments kann dabei manuell durch den Chirurg, manuell mittels eines Manipulators oder automatisch durch einen Roboter erfolgen.

## Patentansprüche

1. Operationssystem zum Durchführen von operativen Eingriffen an einem Patienten (1) mittels eines chirurgischen Instruments (3), das durch einen Roboter oder Manipulator (4) geführt wird, mit
einem Röntgengerät (2) zum Aufnehmen eines Röntgenbildes von mindestens einem Bereich des Patienten (1), wobei das aufgenommene Röntgenbild durch eine Ausgabevorrichtung (11) bildlich dargestellt wird,
einem Positionserfassungssystem zum Erfassen der Position des Röntgengerätes (2) während der Aufnahme und zum permanenten Erfassen der Position des chirurgischen Instruments (3) während des operativen Eingriffs, und
einer Verarbeitungsvorrichtung (10) zum Verarbeiten der erfassten Positionen und zum kontinuierlichen Einblenden einer Darstellung des chirurgischen Instruments (3) in das angezeigte Röntgenbild, wobei die Einblendung des Röntgenbildes und die Darstellung des chirurgischen Instrumentes lagerichtig gemäß der tatsächlichen räumlichen Beziehung zwischen der Position des aufgenommenen Bereichs des Patienten (1) und der Position des chirurgischen Instruments (3) erfolgt,
**dadurch gekennzeichnet,**
**dass** die Verarbeitungsvorrichtung (10) ausgebildet ist, um eine räumliche Position des mit dem Röntgenbild (12) erfassten Bereichs des Patienten aus einer Beziehung zwischen dem Röntgengerät (2) und der in dem Röntgenbild (12) enthaltenen Information, und der mittels des Positionserfassungssystems während der Aufnahme erfassten räumlichen Position des Röntgengerätes (2) zu ermitteln und eine direkte räumliche Beziehung zwischen der ermittelten räumlichen Position des Bereichs des Patienten und der erfassten räumlichen Position des chirurgischen Instruments (3) herzustellen.

2. Operationssystem gemäß Anspruch 1,
**dadurch gekennzeichnet,**
**daß** das Positionserfassungssystem eine erste Positionsmarkierung (5), die an dem Röntgengerät (2) angebracht ist, eine zweite Positionsmarkierung (6), die an dem chirurgischen Instrument (3) angebracht ist, und eine Positionserfassungsvorrichtung (8) zum Erfassen der jeweiligen Positionen von Röntgengerät (2) und dem chirurgischen Instrument (3) aufweist.

3. Operationssystem gemäß Anspruch 1 oder 2,
**gekennzeichnet durch**
eine dritte Positionsmarkierung (7) zum Erfassen der Position des mindestens einen Bereichs des Patienten (1) **durch** die Positionserfassungsvorrichtung (8).

4. Operationssystem gemäß Anspruch 1, 2 oder 3,
**gekennzeichnet durch**
eine Steuervorrichtung zur Steuerung des Roboters (4), wobei der Roboter geeignet ist, einzelne Vorgänge des operativen Eingriffs automatisch durchzuführen.

5. Operationssystem gemäß Anspruch 1, 2 oder 3,
**gekennzeichnet durch**
eine Steuervorrichtung zur Kontrolle eines für den Manipulator vorbestimmten Arbeitsbereichs.

## Claims

1. Operation system for performing operative interventions in a patient (1) by means of a surgical instrument (3) which is guided by a robot or manipulator (4), comprising
an X-ray device (2) for acquiring an X-ray image of at least one area of the patient (1), wherein the acquired X-ray image is pictorially displayed by an output device (11),
a position-recording system for recording the position of the X-ray device (2) during the image acquisition and for permanently recording the position of the surgical instrument (3) during the operative intervention, and
a processing device (10) for processing the recorded positions and for continuously overlaying a representation of the surgical instrument (3) onto the X-ray image displayed, wherein the overlaying of the X-ray image and the representation of the surgical instrument is carried out topographically correctly in accordance with the actual spatial relationship between the position of the imaged area of the patient (1) and the position of the surgical instrument (3),
**characterised in that**
the processing device (10) is fashioned so as to determine a spatial position of the area of the patient recorded in the X-ray image (12) from a relationship between the X-ray device (2) and the information contained in the X-ray image (12) and the spatial position of the X-ray device (2) recorded by means of the position-recording system during the image acquisition and to generate a direct spatial relationship between the determined spatial position of the area of the patient and the recorded spatial position of the surgical instrument (3).

2. Operation system according to Claim 1,
**characterised in that**
the position-recording system has a first position marker (5), which is attached to the X-ray device (2), a second position marker (6), which is attached to the surgical instrument (3), and a position-recording device (8) for recording the respective positions of the X-ray device (2) and the surgical instrument (3).

3. Operation system according to Claim 1 or Claim 2,
**characterised by**
a third position marker (7) for recording the position of at least one area of the patient (1) by means of the position recording device (8).

4. Operation system according to Claim 1, 2 or 3,
**characterised by**
a control device for controlling the robot (4), the robot being suitable for performing individual procedures of the operative intervention automatically.

5. Operation system according to Claim 1, 2 or 3,
**characterised by**
a control device for monitoring a working area predetermined for the manipulator.

## Revendications

1. Système d'opération pour réaliser des interventions opératoires sur un patient (1) à l'aide d'un instrument chirurgical (3) guidé par un robot ou par un manipulateur (4), comportant
- un appareil de radiographie (2) pour prendre une radiographie d'au moins une zone du patient (1), la radiographie prise étant représentée sous forme d'image par un dispositif de présentation (11),
- un système d'enregistrement de la position pour enregistrer la position de l'appareil de radiographie (2) pendant la prise de vue et pour enregistrer en permanence la position de l'instrument chirurgical (3) pendant l'intervention opératoire et
- un dispositif de traitement (10) pour traiter les positions enregistrées et pour incruster en continu une représentation de l'instrument chirurgical (3) sur la radiographie affichée, l'incrustation de la radiographie et la représentation de l'instrument chirurgical étant superposées d'après la position exacte en fonction de la relation spatiale réelle entre la position de la zone relevée du patient (1) et la position de l'instrument chirurgical (3).
**caractérisé en ce que** le dispositif de traitement (10) est conçu
pour établir une position spatiale de la zone du patient enregistrée à l'aide de la radiographie (12) à partir d'une relation entre l'appareil de radiographie (2) et les informations contenues dans la radiographie (12) et la position spatiale de l'appareil de radiographie (2) enregistrée pendant la prise de vue à l'aide du système d'enregistrement de la position et
pour produire une relation spatiale directe entre la position spatiale établie de la zone du patient et la position spatiale enregistrée de l'instrument chirurgical (3).

2. Système d'opération selon la revendication 1, **caractérisé en ce que** le système d'enregistrement de la position présente une première marque de position (5) appliquée à l'appareil de radiographie (2), une deuxième marque de position (6) appliquée à l'instrument chirurgical (3) et un dispositif d'enregistrement de la position (8) pour enregistrer chacune des positions de l'appareil de radiographie (2) et de l'instrument chirurgical (3).

3. Système d'opération selon la revendication 1 ou 2, **caractérisé par** une troisième marque de position (7) pour que le dispositif d'enregistrement de la position (8) enregistre la position de l'au moins une zone du patient (1).

4. Système d'opération selon la revendication 1, 2 ou 3, **caractérisé par** un dispositif de commande pour commander le robot (4), le robot étant conçu pour exécuter des processus particuliers de l'intervention opératoire.

5. Système d'opération selon la revendication 1, 2 ou 3, **caractérisé par** un dispositif de commande pour contrôler une zone de travail prédéfinie pour le manipulateur.
